# EUROPEAN PATENT SPECIFICATION

(11) **EP 4 154 928 B1**
(45) Date of publication and mention of the grant of the patent: **23.09.2026**
(21) Application number: 21809207.0
(22) Date of filing: 06.05.2021
(51) Int. Cl.: A24F 40/20, B05B 11/10, A61M 15/00, A61M 11/00, A24F 42/20, B05B 1/26

(54) **NEBULIZING DEVICE**
VERNEBELUNGSVORRICHTUNG
DISPOSITIF DE NÉBULISATION

(30) Priority: 19.05.2020 CN 202010424951
(43) Date of publication of application: 29.03.2023
(73) Proprietor: Gu, Yu, Jiangsu 215123 (CN)
(72) Inventor: Gu, Yu, Jiangsu 215123 (CN)
(74) Representative: Simmons & Simmons LLP (Munich)
(86) International application number: PCT/CN2021/091920
(87) International publication number: WO 2021/233126

(56) References cited:
- EP-B1- 1 124 603
- WO-A1-2013/080558
- WO-A1-2013/157878
- WO-A1-2020/064340
- CN-A- 1 198 689
- CN-A- 102 186 598
- CN-A- 102 762 309
- CN-A- 103 747 881
- CN-A- 103 917 302
- CN-A- 110 913 935
- CN-U- 203 043 209
- CN-U- 209 378 219
- CN-U- 212 940 910
- JP-A- 2009 538 656
- US-A- 5 370 317
- US-A1- 2012 090 603
- US-A1- 2014 203 049
- US-A1- 2016 095 992

## Description

### FIELD OF THE INVENTION

This application relates to the field of nebulizing devices, and more specifically, to a nebulizing device mainly used for pulmonary inhalation.

### BACKGROUND

A nebulizing device used for pulmonary inhalation mainly converts a liquid preparation such as drugs, nutrients, or nicotine liquid into tiny particles that can be inhaled into a human through breathing, so as to achieve effective delivery of the liquid preparation. Currently, common nebulizing devices used for pulmonary inhalation include a metered dose inhaler (MDI), a nebulizer (Nebulizer), a soft mist inhaler (SMI), a dry powder inhaler (DPI), and the like. However, all these devices have certain problems and defects.

A conventional mechanical nebulizing device uses a firing pre-compression spring to spray liquid of a specified volume from a liquid channel to the outside through a nozzle, so as to obtain an aerosol. However, such a nebulizing device usually requires a user to manually rotate upper and lower housings of the nebulizing device to realize addition of medicinal liquid from a reservoir to the liquid channel, and simultaneously realize pre-compression of the spring. However, this manual operation lacks feedback to remind the user of an operation speed, which may lead to an inaccurate volume of the added medicinal liquid.

The following documents are known in the art, namely US 5 370 317 A, US 2016/095992 A1, US 2014/203049 A1 and EP 1 124 603 B1.

Therefore, it is necessary to provide an improved nebulizing device for pulmonary inhalation.

### SUMMARY

The invention is defined in the appended claims. This application is directed to provide an improved nebulizing device for pulmonary inhalation.

According to an aspect of this application, a nebulizing device is provided, including: a hollow housing, where the hollow housing includes a cavity, and the cavity includes a liquid channel located inside the cavity and a nebulizing spray nozzle located at a distal end of the liquid channel; and an injector, where the injector is at least partially accommodated in the cavity, and coupled to the hollow housing through a bias spring, and the injector includes: a reservoir, configured to accommodate liquid; a liquid conveying pipe, including a liquid inlet and a liquid outlet arranged opposite to each other, where the liquid inlet is located in the reservoir, the liquid outlet is located in the liquid channel, and the liquid outlet and the nebulizing spray nozzle jointly define a liquid chamber; and an injector proximal end, coupled to the liquid conveying pipe and configured to operatively receive a pushing force, where under an action of the pushing force and the bias spring, the liquid conveying pipe is movable between a proximal position in the cavity and a distal position relatively closer to the distal end; and when the liquid conveying pipe is at the distal position, the bias spring is in a compressed state, where when the pushing force is removed from the injector proximal end, the bias spring is capable to drive the liquid conveying pipe to move from the distal position to the proximal position, so as to convey the liquid from the reservoir into the liquid chamber; and when the injector proximal end receives the pushing force, the pushing force is capable to compress the bias spring to drive the liquid conveying pipe to move from the proximal position to the distal position, so as to form a predetermined nebulizing pressure at the nebulizing spray nozzle, so that the liquid in the liquid chamber can flow out of the liquid channel through the nebulizing spray nozzle.

According to another aspect of this application, a nebulizing device is provided, including: a hollow housing, where the hollow housing includes a cavity, and the cavity includes a liquid channel located inside the cavity and a nebulizing spray nozzle located at a distal end of the liquid channel, where the nebulizing spray nozzle includes a plurality of fluid outlets; and an injector, where the injector is at least partially accommodated in the cavity, and the injector includes: a reservoir, configured to accommodate liquid; a liquid conveying pipe, including a liquid inlet and a liquid outlet arranged opposite to each other, where the liquid inlet is located in the reservoir, the liquid outlet is located in the liquid channel, the liquid outlet and the nebulizing spray nozzle jointly define a liquid chamber, and the liquid conveying pipe is configured to operatively convey the liquid accommodated in the reservoir into the liquid chamber; and an injector proximal end, where the injector proximal end is coupled to the liquid conveying pipe and configured to receive a pushing force, and under an action of the pushing force, the liquid conveying pipe is movable in the cavity from a proximal position to a distal position relatively closer to the distal end to form a predetermined nebulizing pressure at the nebulizing spray nozzle, so that the liquid in the liquid chamber can flow out of the liquid channel through the plurality of fluid outlets of the nebulizing spray nozzle and converge to generate a plume spray with a predetermined initial speed, where at least 50% of droplets in the plume spray have an average particle size ranging from 1 um to 10 um.

In some embodiments, at least 50% of the droplets in the plume spray have an average particle size ranging from 2 um to 5 um.

In some embodiments, the predetermined initial speed is less than 10 m/s.

In some embodiments, the predetermined initial speed ranges from 1 m/s to 5 m/s.

In some embodiments, during generation of the plume spray, the pushing force is non-monotonically attenuated.

In some embodiments, the nebulizing device further includes a bias spring; the injector is coupled to the hollow housing through the bias spring; and when the liquid conveying pipe is at the distal position, the bias spring is in a compressed state, and when the pushing force is removed from the injector proximal end, the bias spring is capable to drive the liquid conveying pipe to move from the distal position back to the proximal position, so as to convey the liquid from the reservoir into the liquid chamber.

In some embodiments, the bias spring is configured to, when the pushing force pushes the liquid conveying pipe to move from the proximal position to the distal position, generate a resistance in an opposite direction to the pushing force, and the resistance is used for substantially offsetting an increased pushing force, so that the predetermined nebulizing pressure is maintained at the nebulizing spray nozzle.

In some embodiments, the bias spring is configured to generate a damping when the pushing force pushes the liquid conveying pipe to move from the proximal position to the distal position, and the damping and a liquid damping in the liquid channel cause a time for the liquid conveying pipe to move from the proximal position to the distal position to be not less than a predetermined nebulizing time.

In some embodiments, the predetermined nebulizing time ranges from 0.5 seconds to 5 seconds.

In some embodiments, the damping causes the time for the liquid conveying pipe to move from the proximal position to the distal position to be substantially equal to a single inhalation time of a normal breathing rhythm of a human.

In some embodiments, the pushing force is manually applied.

In some embodiments, a magnitude of the pushing force ranges from 10 N to 70 N.

In some embodiments, the nebulizing device further includes a distal stopper, and the distal stopper is arranged in the hollow housing and is configured to, when the liquid conveying pipe is at the distal position, prevent the liquid conveying pipe from moving distally.

In some embodiments, the nebulizing device further includes a proximal stopper, and the proximal stopper is arranged in the hollow housing and is configured to, when the liquid conveying pipe is at the proximal position, prevent the liquid conveying pipe from moving proximally.

In some embodiments, a total cross-sectional area of the plurality of fluid outlets of the nebulizing spray nozzle ranges from 20 um² to 1000 um².

In some embodiments, the predetermined nebulizing pressure ranges from 80 MPa to 1000 MPa.

In some embodiments, the predetermined initial speed ranges from 0.5 m/s to 2 m/s.

In some embodiments, the initial speed of the plume spray generated during movement of the liquid conveying pipe from the proximal position to the distal position is substantially maintained between 1 m/s to 5 m/s for a continuous time period of 40% to 80% of a plume spray duration.

In some embodiments, the initial speed of the plume spray generated during movement of the liquid conveying pipe from the proximal position to the distal position remains substantially constant for a continuous time period of 40% to 80% of a plume spray duration.

In some embodiments, in the continuous time period of the substantially constant initial speed of the plume spray, at least 50% of the droplets have an average particle size ranging from 1 um to 10 um.

In some embodiments, when the liquid conveying pipe is at the proximal position, the bias spring is substantially in a relaxation state or in an under-compression state.

The above is a summary of this application, and there may be cases of simplification, generalization, and omission of details, so that a person skilled in the art should realize that this part is only illustrative, and is not intended to limit the scope of this application in any way. This summary is not intended to identify key features or essential features of the claimed subject matter, nor is it intended to be used as an aid in determining the scope of the claimed subject matter.

### BRIEF DESCRIPTION OF THE DRAWINGS

The above and other features of content of this application will be more fully and clearly understood from the following description and appended claims, in combination with the accompanying drawings. It may be understood that these accompanying drawings depict only several implementations of the content of this application and should not be considered as limiting the scope of the content of this application. The content of this application will be explained more clearly and in detail through the use of the accompanying drawings.
FIG. 1 shows a schematic diagram of a nebulizing device 100 when a liquid conveying pipe thereof is at a proximal position according to an embodiment of this application;
FIG. 2 shows a schematic diagram of the nebulizing device 100 described in FIG. 1 when the liquid conveying pipe thereof is at a distal position;
FIG. 3 shows an exploded view of the nebulizing device 100 shown in FIG. 1;
FIG. 4 shows a schematic cross-sectional view of a nebulizing spray nozzle 103 of the nebulizing device 100 shown in FIG. 1 to FIG. 3; and
FIG. 5 shows a schematic use flowchart of the nebulizing device 100 shown in FIG. 1 to FIG. 4 delivering a liquid preparation to a lung in coordination with a breathing rhythm of a human.

### DETAILED DESCRIPTION

In the following detailed description, reference is made to the accompanying drawings which form a part hereof. In the accompanying drawings, similar symbols generally represent similar components, unless the context dictates otherwise. The detailed description, the accompanying drawings, and the illustrative implementations described in the claims are not intended to limit. Without departing from the scope of the subject matter of this application, other implementations may be adopted and other changes may be made.

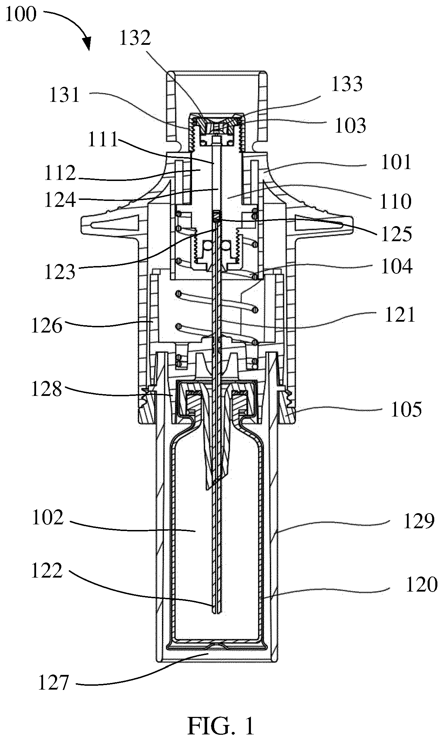

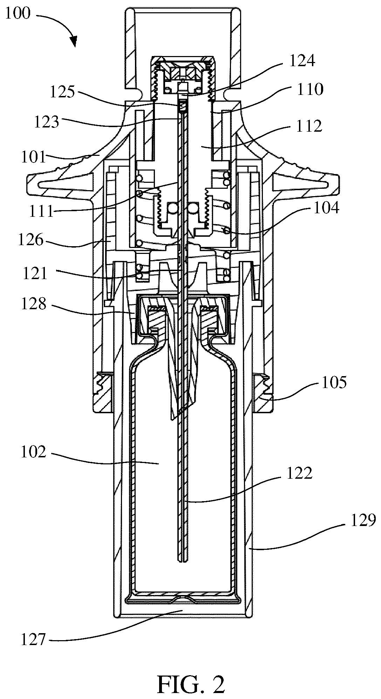

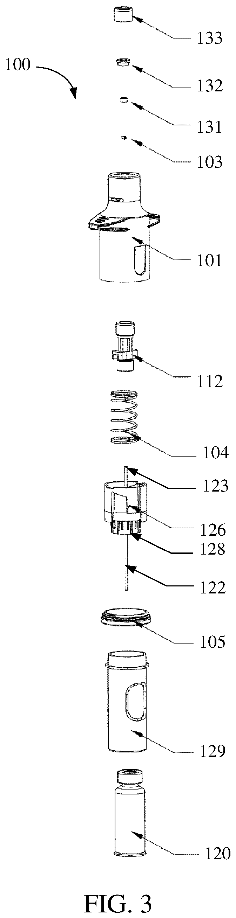

FIG. 1 is a schematic diagram of a nebulizing device 100 when a liquid conveying pipe thereof is at a proximal position according to an embodiment of this application. FIG. 2 is a schematic diagram of the nebulizing device 100 when the liquid conveying pipe thereof is at a distal position. FIG. 3 shows an exploded view of the nebulizing device 100. The liquid conveying pipe of the nebulizing device 100 is movable between the proximal position and the distal position shown in the figures, so as to realize a circulation of liquid injection-nebulizing-liquid injection.

As shown in FIG. 1 to FIG. 3, the nebulizing device 100 includes a hollow housing 101 and an injector 102. The hollow housing 101 includes a cavity 110, the injector 102 is at least partially accommodated in the hollow housing 101, and the injector can reciprocate between the proximal position shown in FIG. 1 and the distal position shown in FIG. 2. In some embodiments, as shown in FIG. 3, the injector 102 of the nebulizing device 100 may include a peripheral portion 126, a shape of which is configured to substantially match a cross-sectional shape of the cavity 110 of the hollow housing 101, so that the injector 102 can reciprocate in the cavity 110 along an axial direction of the nebulizing device 100.

Still referring to FIG. 1 to FIG. 3, the cavity 110 includes a liquid channel 111 located inside the cavity. The liquid channel 111 may be configured to accommodate liquid to be nebulized. The liquid channel 111 shown in the figures has substantially a same cross section along an axial direction of the liquid channel. In some embodiments, a size of the cross section of the liquid channel 111 is set to range from 0.5 mm² to 10 mm². In some other embodiments, the liquid channel 111 may have a variable cross-sectional area, for example, the liquid channel 111 has a segmented structure, which has two or more different cross-sectional areas at different axial positions. In some embodiments, the liquid channel 111 has a circular cross section, but in some other embodiments, the cross section may also be set to other shapes, such as a rectangle or an ellipse. With reference to FIG. 1 and FIG. 3, the liquid channel 111 is defined by a liquid guide member 112 arranged in the cavity 110 of the hollow housing 101. In some embodiments, the liquid guide member 112 is detachably fixed to the hollow housing 101, while in some other embodiments, the liquid guide member 112 may be integrally formed with the hollow housing 101.

As shown in FIG. 1 and FIG. 2, a distal end of the liquid channel 111 is provided with a nebulizing spray nozzle 103, and the nebulizing spray nozzle 103 may be provided with one or more fluid outlets, preferably, a plurality of fluid outlets. Under an action of a pressure in the liquid channel 111, a liquid preparation accommodated in the liquid channel 111 can be sprayed out through the plurality of fluid outlets on the nebulizing spray nozzle 103, and converge to finally form nebulized particles of the liquid preparation. Specific settings of the fluid outlets on the nebulizing spray nozzle 103 are described in detail below.

Referring to FIG. 1 and FIG. 3, the nebulizing spray nozzle 103 is arranged at the distal end of the liquid channel 111 in the liquid guide member 112, and a nozzle protection member 131, a nozzle pressing member 132, and a nozzle fastening member 133 are arranged on the nebulizing spray nozzle 103 in sequence. The nozzle protection member 131 is configured to protect the nebulizing spray nozzle 103 and fix the nebulizing spray nozzle 103, the nozzle pressing member 132 is configured to press and stabilize the nozzle protection member 131, and the nozzle fastening member 133 is configured to fasten the nozzle pressing member 132 to the liquid guide member 112. In some embodiments, the nozzle fastening member 133 fixes the nebulizing spray nozzle 103 to the distal end of the liquid channel 111 of the liquid guide member 112 through threaded connection to the liquid guide member 112. In some other embodiments, the nozzle fastening member 133 may also fix and attach the nebulizing spray nozzle 103 to the liquid guide member 112 or the hollow housing in other fastening manners, for example, snap-in connection, adhesive connection, or the like. It should be noted that, in some embodiments, the nebulizing device 100 may only include any one or two of the nozzle protection member 131, the nozzle pressing member 132, and the nozzle fastening member 133. For example, the nebulizing spray nozzle 103 itself may have a corresponding threaded or snap-in structure to fasten the nebulizing spray nozzle 103 to the liquid guide member 112. In some embodiments, the nozzle pressing member 132 or the nozzle fastening member 133 may be integrally formed with the liquid guide member 112.

Still referring to FIG. 1 to FIG. 3, the injector 102 includes a reservoir 120 configured to accommodate liquid and a liquid conveying pipe 121. The liquid conveying pipe 121 includes a liquid inlet 122 and a liquid outlet 123 arranged opposite to each other, the liquid inlet 122 is arranged in the reservoir 120 and generally located at a proximal position close to the reservoir 120, and the liquid outlet 123 is located in the liquid channel 111. The liquid outlet 123 and the liquid channel 111 jointly define a liquid chamber 124, so that the liquid accommodated in the reservoir 120 can operatively flow into the liquid chamber 124 through the liquid conveying pipe 121. Under an action of a pressure difference between the liquid chamber 124 and the reservoir 120 (in this case, a pressure of the liquid chamber 124 is lower than a pressure of the reservoir 120), the liquid accommodated in the reservoir 120 flows into the liquid chamber 124 through the liquid conveying pipe 121, so as to realize addition of the liquid preparation, that is, liquid injection. As shown in FIG. 1 and FIG. 2, the liquid outlet 123 is further provided with a one-way valve member 125, so that the liquid can flow into the liquid chamber 124 through the one-way valve member 125, but cannot flow out of the liquid chamber 124 in an opposite direction.

The nebulizing device 100 further includes a bias spring 104 coupled between the injector 102 and the hollow housing 101, so that the injector 102 is coupled to the hollow housing 101 through the bias spring 104. Specifically, a distal end of the bias spring 104 abuts against the liquid guide member 112 in the cavity 110, and a proximal end of the bias spring abuts against the peripheral portion 102 of the injector 126. When the liquid conveying pipe 121 is at the proximal position shown in FIG. 1, the bias spring 104 is substantially in a relaxation state or in an under-compression state. When a pushing force applied to an injector proximal end 127 pushes the injector 102 to move to the distal end, to cause the liquid conveying pipe 121 to move to the distal position shown in FIG. 2, the bias spring 104 is in a compressed state. The under-compression state herein refers to that a compression amount of the bias spring 104 is less than the compression amount of the bias spring at the distal position. In this case, if the pushing force is removed from the injector proximal end 127, the bias spring 104 can drive the liquid conveying pipe 121 to move from the distal position back to the proximal position. With movement of the liquid conveying pipe from the distal position to the proximal position, the pressure in the liquid chamber 124 is reduced, so that the liquid in the reservoir 120 can be conveyed from the reservoir 120 through the liquid conveying pipe 121 to the liquid chamber 124 under the action of the pressure difference.

Still referring to FIG. 1 to FIG. 3, the nebulizing device 100 further includes a distal stopper, and the distal stopper is arranged in the hollow housing 101 and is configured to, when the liquid conveying pipe 121 is at the distal position shown in FIG. 2, prevent the liquid conveying pipe 121 from moving distally. In some embodiments, the distal stopper is arranged on the liquid guide member 112, and is configured to, when the liquid conveying pipe 121 is at the distal position, abut against a corresponding structure or part on the injector 102, to prevent the liquid conveying pipe 121 from moving distally. In some other embodiments, the distal stopper is arranged on an inner wall of the cavity 110 of the hollow housing 101, and is configured to, when the liquid conveying pipe 121 is at the distal position, abut against a corresponding structure or member on the peripheral portion 126 of the injector 102, to prevent the liquid conveying pipe 121 from moving distally.

Similarly, the nebulizing device 100 further includes a proximal stopper 105, and the proximal stopper 105 is arranged in the hollow housing 101 and is configured to, when the liquid conveying pipe 121 is at the proximal position shown in FIG. 1, prevent the liquid conveying pipe 121 from moving proximally. Specifically, as shown in FIG. 1 to FIG. 3, the proximal stopper 105 is an end cap of the proximal end inserted into the hollow housing 101. When the liquid conveying pipe 121 is at the proximal position, the proximal stopper abuts against a protrusion structure on a periphery of a proximal housing 129 of the injector 102, to prevent the liquid conveying pipe 121 from moving proximally. In some embodiments, the proximal stopper 105 may also be arranged on a protrusion or a similar structure on an inner wall of the hollow housing 101, and match a corresponding locking structure on a periphery of the injector 102, to prevent the liquid conveying pipe 121 from moving proximally. It may be understood that, in some examples, when the bias spring 104 is at the proximal position, the bias spring is in a relaxation state; and correspondingly, the proximal stopper does not need to be arranged on the nebulizing device 100.

It can be seen that, a length of the bias spring 104 can vary between a liquid injection length defined by the proximal stopper 105 and a release length defined by the distal stopper. In this case, the addition of the liquid preparation in the liquid chamber 124 depends on an action of the bias spring 104, and mechanical energy of the bias spring accumulated at the distal position can be used for forming the pressure difference between the reservoir 120 and the liquid chamber 124. During an addition process of the liquid preparation, a user does not need to operate the nebulizing device 100, so that each liquid injection operation can stably add a liquid preparation of a predetermined volume into the liquid chamber 124. This structural design of automatic liquid injection also avoids uncertainty caused by a manual operation.

In some embodiments, during a nebulizing process, a movement stroke of the liquid conveying pipe 121 and a cross section of an inner cavity of the liquid chamber 124 jointly determine a single dose. In some embodiments, a diameter of the cross section of the inner cavity ranges from 1 mm to 5 mm, the movement stroke ranges from 5 mm to 30 mm, and the single dose ranges from 1 µL to 500 µL. In some preferred embodiments, the diameter of the cross section of the inner cavity ranges from 1 mm to 3 mm, the movement stroke ranges from 5 mm to 20 mm, and the single dose ranges from 2 µL to 150 µL.

In some embodiments, the reservoir 120 is detachably engaged with the liquid conveying pipe 121, so that the reservoir 120 can be replaced or refilled after the liquid preparation accommodated in the reservoir are used up. Specifically, as shown in FIG. 3, a periphery of the liquid conveying pipe 121 is provided with a proximal end engaging portion 128, and the proximal end engaging portion 128 has a clamping groove or snap structure matching a shape of the distal end of the reservoir 120, so as to be detachably engaged with the reservoir 120. It should be noted that, the proximal end engaging portion 128 may also be arranged as other common engaging structures to be detachably engaged with the reservoir 120. In some embodiments, the liquid conveying pipe 121 and the reservoir 120 may be integrally formed.

Still referring to FIG. 1 and FIG. 2, the injector 102 includes the injector proximal end 127, and the injector proximal end 127 is coupled to the liquid conveying pipe 121, so that the pushing force applied to the injector proximal end 127 can overcome a force applied by the bias spring and drive the liquid conveying pipe 121 to move from the proximal position shown in FIG. 1 to the distal position shown in FIG. 2. The movement of the liquid conveying pipe 121 to the distal end can generate a pressure in the liquid channel 111, and generate a sufficient predetermined nebulizing pressure at the nebulizing spray nozzle 103, so that the liquid preparation accommodated in the liquid chamber 124 is under pressure and sprayed through the nebulizing spray nozzle 103. It may be understood that, the predetermined nebulizing pressure at the nebulizing spray nozzle described herein refers to a liquid pressure when the liquid preparation enter the nebulizing spray nozzle, and the liquid pressure causes the liquid preparation having energy to enter the nebulizing spray nozzle and being sprayed to form nebulized droplets.

It should be noted that, in some embodiments, the injector proximal end 127 may also be any other position or part to which a force can be applied on the injector 102 that is relatively close to the proximal end, and the injector proximal end 127 is constructed to be suitable for applying a pushing force to drive the liquid conveying pipe 121 to move between the proximal position and the distal position. In some embodiments, the injector proximal end 127 and the nebulizing device 100 are constructed to be suitable for accepting a manually applied force, such as a pushing force applied by a thumb, an index finger, or another hand region. In some embodiments, the injector proximal end 127 and the nebulizing device 100 are constructed to be suitable for accepting a pushing force ranging from 10 N to 70 N, which is substantially comparable to a pushing force applied by an ordinary person when grasping the nebulizing device 100 in a clamping or holding posture. It may be understood that the foregoing range of 10 N to 70 N varies depending on abilities, experience, and/or usage habits of different people, but generally, a force applied by each person during use is relatively stable and does not vary greatly within this range. In some other embodiments, the injector proximal end 127 is constructed to be able to engage with an automatic force application device to receive an automatically applied force. For example, the automatic force application device may be constructed to include a motor and a power supply, and the motor can provide a pushing force along an axial direction of the injector 102 under driving of the power supply.

Specifically, during a generation process of a plume spray, the liquid conveying pipe 121 moves from the proximal position to the distal position, so that a volume of the liquid chamber 124 is reduced and the pressure is increased to form a pressure in the liquid chamber 124, and the pressure further generates the predetermined nebulizing pressure at the nebulizing spray nozzle 103, to cause the liquid preparation in the liquid chamber 124 to flow out of the liquid channel 111 through the plurality of fluid outlets on the nebulizing spray nozzle 103 and converge, to finally form a plume spray with a predetermined initial speed and/or particle size. In some embodiments, the nebulizing device 100 is constructed as that, under common use, the predetermined nebulizing pressure formed by the movement of the liquid conveying pipe 121 at the nebulizing spray nozzle 103 ranges from 80 MPa to 1000 MPa, preferably ranges from 100 MPa to 600 MPa, and more preferably ranges from 300 MPa to 500 MPa. A person skilled in the art may understand that, structures of the liquid channel 111 and the nebulizing spray nozzle 103 may be designed to cooperate with the liquid nebulizing pressure to form a liquid preparation plume spray with required properties. The properties of the liquid preparation plume spray may include an initial speed (a speed as the plume spray leaves the nebulizing spray nozzle) and/or an average particle size. In general, under a given pressure, a pore size of the nebulizing spray nozzle, a filter structure (such as a filter column or filter element) upstream of the nebulizing spray nozzle in the liquid channel, a length and cross-sectional area of the liquid channel, a viscosity of the liquid preparation, and the like all affect the initial speed and the average particle size, and a person skilled in the art may design and determine these structure parameters according to theoretical calculation and simulation results of fluid mechanics.

In some embodiments, the nebulizing device 100 is constructed as that, under common use, an initial speed of the generated liquid preparation plume spray is less than 10 m/s, and preferably ranges from 1 m/s to 5 m/s or ranges from 0.5 m/s to 2 m/s. In some embodiments, the nebulizing device 100 is constructed as that, under common use, at least 50% of droplets in the generated plume spray have an average particle size ranging from 1 um to 10 um, and preferably have an average particle size ranging from 2 um to 5 um. The plume spray with the initial speed and the average particle size can be easily inhaled by a human respiratory tract and can be adequately absorbed in the lung, thereby effectively improving bioavailability efficiency. In addition, an amount of the formed plume spray exhaled again out of the body through a breathing movement of the human is relatively small, which is conducive to reducing pollution of the liquid preparation to the environment.

In some embodiments, the nebulizing device 100 is constructed as that, under common use, a time for the liquid conveying pipe 121 to move from the proximal position to the distal position is substantially equal to a single inhalation time of a normal breathing rhythm of a human. In some embodiments, the nebulizing device 100 is constructed as that, under common use, a duration of a plume spray formed during the movement of the liquid conveying pipe 121 from the proximal position to the distal position is substantially equal to a single inhalation time of a normal breathing rhythm of a human. In this way, the plume spray formed during the movement from the proximal position to the distal position can substantially match an inhalation action of a human, so that the plume spray can be effectively inhaled into the human, to avoid a case that the liquid preparation is wasted or most of the liquid preparation cannot be inhaled by the human, and reduce an amount of unabsorbed plume spray that is exhaled into the environment. In some embodiments, the nebulizing device 100 is constructed as that, under common use, the duration of the plume spray formed during the movement of the liquid conveying pipe 121 from the proximal position to the distal position, that is, a nebulizing time, ranges from 0.5 seconds to 5 seconds, and preferably ranges from 1 second to 3 seconds. It may be understood that, on one hand, the plume spray duration depends on factors such as an amount of liquid preparations consumed by one spraying and a movement distance of the liquid conveying pipe 121, and on the other hand, the duration also depends on fluid characteristics (for example, a liquid damping) of the liquid channel of the nebulizing device 100. A person skill in the art may adjust related designs and parameters according to an actual requirement to adjust the duration of the plume spray. It should be noted that, since the liquid in the liquid channel during a spraying process has a liquid damping, in a case that the amount of the liquid preparations consumed by one spraying is given, impact of a pushing force to the injector proximal end on a moving speed of the liquid conveying pipe 121 is relatively small. In other words, a large range of changes of the pushing force does not cause an equivalent range (in proportion) of changes of the duration, which helps improve the operation stability of the nebulizing device 100 in a spraying state.

In some embodiments, the nebulizing device 100 is constructed as that, under common use, the initial speed of the plume spray formed during the movement of the liquid conveying pipe 121 from the proximal position to the distal position remains ranging from 1 m/s to 5 m/s or from 0.5 m/s to 2 m/s. In some embodiments, the nebulizing device 100 is constructed as that, under common use, the initial speed of the plume spray formed during the movement of the liquid conveying pipe 121 from the proximal position to the distal position remains basically constant. In some embodiments, the nebulizing device 100 is constructed as that, under common use, a plume spray formed during the movement of the liquid conveying pipe 121 from the proximal position to the distal position can substantially remains the above-mentioned initial speed for a continuous time period of 40% to 80%, and preferably for a continuous time period of 60% to 80% of the plume spray duration, for example, remains ranging from 1 m/s to 5 m/s or from 0.5 m/s to 2 m/s, or substantially constant. In some embodiments, the nebulizing device 100 is constructed as that, under common use, in the plume spray formed during the above-mentioned at least 40% to 80% continuous time period, at least 50% of droplets have an average particle size ranging from 1 um to 10 um, and preferably, an average particle size ranging from 2 um to 5 um.

The initial speed and the average particle size of the plume spray mainly depend on characteristics of the liquid channel of the nebulizing device, especially a structure and design of the most downstream nebulizing spray nozzle and a liquid pressure of the liquid preparation when entering the nebulizing spray nozzle.

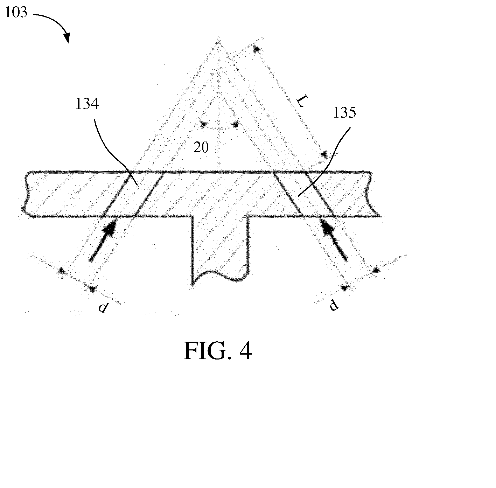

FIG. 4 shows a schematic cross-sectional view of a nebulizing spray nozzle 103 of the nebulizing device 100 shown in FIG. 1 to FIG. 3. As shown in FIG. 4, the nebulizing spray nozzle 103 includes fluid outlets 134 and 135, orientations of the fluid outlets are arranged to cause liquid flowing out of the nebulizing spray nozzles can converge, to generate a plume spray with a predetermined initial speed and an average particle size. In some embodiments, the nebulizing spray nozzle 103 and the nebulizing device 100 are constructed as that, under common use, the generated plume spray can have the ideal properties mentioned above, such as the range of the initial speed, the average particle size of at least 50% of the droplets, and the duration of the stable initial speed. Specifically, in some embodiments, a total cross-sectional area of the plurality of fluid outlets 134 and 135 ranges from 20 um² to 1000 um², preferably ranges from 50 um² to 500 um², and more preferably ranges from 50 um² to 300 um².

Cross sections of the liquid channel 111 and the fluid outlets 134 and 135 of the nebulizing device 100 may be in a shape of a circle, a rectangle, or any other suitable shape. In some embodiments, the cross sections of the fluid outlets 134 and 135 are in a shape of a circle, and a diameter of the circle ranges from 5.5 um to 26 um. Still referring to FIG. 4, inlet angles of the fluid outlets 134 and 135 are θ, and an angle formed by orientations of the fluid outlets is 2θ. Specifically, in some embodiments, the inlet angle θ of the fluid outlets 134 and 135 ranges from 15 degrees to 75 degrees, and preferably ranges from 30 degrees to 60 degrees. According to the above construction combined with other constructions of the nebulizing device 100, under common use, a plume spray formed by the liquid preparation flowing out of the nebulizing spray nozzle 103 can have an initial speed and an average particle size more suitable for pulmonary inhalation.

It should be noted that, although the nebulizing spray nozzle 103 shown in FIG. 4 includes the fluid outlets 134 or 135 arranged opposite to each other, in some embodiments, the nebulizing spray nozzle 103 may alternatively include three, four, five, six, or more fluid outlets. These fluid outlets may be uniformly distributed on the nebulizing spray nozzle 103. Specifically, in some embodiments, at least one or several pairs of the fluid outlets can form the inlet angle, a cross-sectional area, and/or a length-diameter ratio of the fluid outlet 134 or 135 mentioned above.

It may be understood that the structure design of the nebulizing spray nozzle is only exemplary, and a person of ordinary skill in the art may adjust the structure and parameters of the nebulizing spray nozzle and structure and design parameters of other components in the liquid channel according to a nebulizing requirement of the liquid preparation.

In some embodiments, the liquid preparation to be conveyed is a drug, a nutrient, or a liquid preparation containing nicotine substances, and a viscosity thereof ranges from 0.2 mPa·s to 1.6 mPa·s at 25°C as measured by an Ostwald viscometer. In some embodiments, the viscosity of the liquid preparation ranges from 0.5 mPa•s to 1.3 mPa•s, and preferably ranges from 0.8 mPa•s to 1.1 mPa•s at 25°C as measured by the Ostwald viscometer.

It should be further noted that "under common use" described herein refers to a case that under a common room temperature environment, the foregoing pushing force, such as 30 N to 50 N, is applied to the nebulizing device 100, and the liquid preparation to be conveyed has the Ostwald viscosity as described above. The "proximal end" and "distal end" mentioned herein refer to an end relatively far away from the nebulizing spray nozzle 103 and an end relatively close to the nebulizing spray nozzle 103.

As shown in FIG. 1 to FIG. 3, in some embodiments, the injector 102 further includes a proximal housing 129, and the proximal housing 129 is arranged around the reservoir 120 to effectively protect the reservoir 120. An opening is provided on the proximal housing 129, to facilitate a user to observe properties and an inventory of the liquid in the reservoir 120 through the opening. In some embodiments, the proximal housing 129 is at least partially set to be transparent or unobstructed. In some other embodiments, scales are correspondingly provided on the proximal housing 129 or the reservoir to facilitate to observe the properties and the inventory of the liquid in the reservoir 120.

In some embodiments, during a plume spray generation duration of the nebulizing device 100, that is, during a movement process of the liquid conveying pipe 121 from the proximal position to the distal position, the pushing force applied to the injector proximal end 127 is non-monotonically attenuated. In some embodiments, the pushing force is gradually increased. The bias spring 104 is configured to, under common use, when the pushing force drives the liquid conveying pipe 121 to move from the proximal position shown in FIG. 1 to the distal position shown in FIG. 2, be able to generate a resistance in an opposite direction to the pushing force, and the resistance is used for substantially offsetting an increased pushing force, so that the predetermined nebulizing pressure is maintained at the nebulizing spray nozzle. In some embodiments, the predetermined nebulizing pressure ranges from 80 MPa to 1000 MPa, preferably ranges from 100 MPa to 600 MPa, and more preferably ranges from 300 MPa to 500 MPa. In a case that the pushing force is applied manually, due to a common force application habit of a human, after the pushing force is applied, the pushing force applied by the user to press the injector proximal end is generally gradually increased, and the setting of the bias spring 104 exactly can effectively offset the gradually increasing part of the pushing force, so that a stable nebulizing pressure is maintained at the nebulizing spray nozzle, so as to form a continuous plume spray with a substantially constant initial speed and average particle size.

In some embodiments, the bias spring 104 is configured to, under common use, when the liquid conveying pipe 121 is pushed to move from the proximal position shown in FIG. 1 to the distal position shown in FIG. 2, a damping is generated, and the damping and a liquid damping in the liquid channel cause a time for the liquid conveying pipe 121 to move from the proximal position to the distal position to be not less than a predetermined nebulizing time. In some embodiments, the predetermined nebulizing time ranges from 0.5 seconds to 5 seconds, and preferably ranges from 1 second to 3 seconds. In some embodiments, the bias spring 104 is configured to, under common use, when the liquid conveying pipe 121 moves from the proximal position to the distal position, a damping is generated, and the damping and the liquid damping cause a duration of a plume spray formed by the nebulizing device 100 to be substantially equal to a single inhalation time of a normal breathing rhythm of a human. In some embodiments, the bias spring 104 is configured to, under common use, when the liquid conveying pipe 121 moves from the proximal position to the distal position, a damping is generated, and the damping and the liquid damping cause the duration of the plume spray formed by the nebulizing device 100 to range from 0.5 seconds to 5 seconds, and preferably range from 1 second to 3 seconds. Due to the existence of the bias spring 104, under common use, a moving speed of the liquid conveying pipe 121 is within a certain range, so that the duration of the formed plume spray is substantially consistent with the inhalation time. Therefore, a utilization rate of the liquid preparation can be effectively improved to avoid unnecessary waste and pollution.

In a conventional mechanical nebulizing device, liquid nebulizing is generally realized by firing a bias spring. During a firing and release process of the bias spring, a compression amount of the bias spring may be gradually decreased, so that a nebulizing pressure that the bias spring can provide is also gradually decreased. However, the conventional mechanical nebulizing device intends to always realize nebulizing of the liquid preparation throughout the entire nebulizing process, so that the nebulizing device is designed to be capable to generate an adequate nebulizing pressure even at a position where the compression amount of the bias spring is minimum. Correspondingly, in a case that the bias spring has a relatively large compression amount at another position, the nebulizing device actually has redundancy, and due to the existence of the redundancy, a particle size and an initial speed of a liquid preparation plume spray formed through nebulizing are not always satisfactory.

Compared with the conventional mechanical nebulizing device, the pushing force of the nebulizing device in some embodiments of this application is manually applied by the user, and the force application habit of a human causes the manually applied pushing force to be able to form a nebulizing pressure with relatively small changes at the nebulizing spray nozzle, so as to form a continuous plume spray with a substantially constant initial speed and average particle size. This is far superior to the conventional mechanical nebulizing device.

In addition, a firing process of the conventional mechanical nebulizing device is short, and compression energy of the spring is released too quickly, which accelerates metal fatigue of the spring and reduce a service life of the nebulizing device. On the contrary, the nebulizing device according to the embodiments of this application can be manually pushed by the user, and medicinal liquid can be released relatively slowly, which helps reduce wear and tear of the spring and improve the service life. In addition, rapid energy release causes the conventional mechanical nebulizing device incapable to convey a relative large amount of liquid preparations (usually only 10 microliters to 30 microliters of liquid can be conveyed), so that many requirements for large-dose drug administration cannot be met. On the contrary, the nebulizing device according to the embodiments of this application can be continuously administered by the user for multiple times, which can be suitable for administration of large-dose liquid preparations (for example, 1 mL or more of liquid preparations can be conveyed). In addition, as described above, under an operation of multiple consecutive administration, the nebulizing device according to the embodiments of this application can perform administration in accordance with a breathing rhythm of a human, which is conducive to improving inhalation efficiency of the liquid preparation.

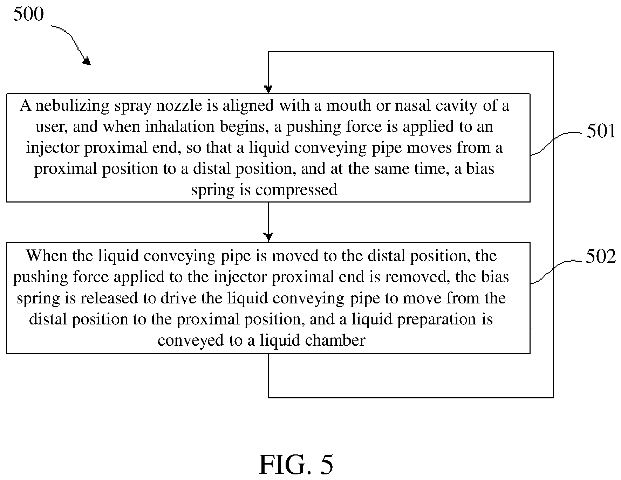

FIG. 5 shows a schematic use flowchart of the nebulizing device 100 shown in FIG. 1 to FIG. 3 delivering a liquid preparation to a lung in coordination with a breathing rhythm of a human. The following describes a process of using the nebulizing device 100 to match a breathing rhythm of a human to deliver the above-mentioned liquid preparation containing nicotine substances with the Ostwald viscosity to a lung with reference to FIG. 5. First, in step 501, the nebulizing spray nozzle 103 is aligned with a mouth or nasal cavity of a user, and when inhalation begins, a pushing force is applied to the injector proximal end 127, so that the liquid conveying pipe 121 moves from the distal position shown in FIG. 1 to the proximal position shown in FIG. 2. In this process, the bias spring is compressed. With the movement of the liquid conveying pipe 121, a predetermined nebulizing pressure is generated at the nebulizing spray nozzle 103, so that the liquid containing nicotine substances in the liquid chamber 124 can flow out from the fluid outlets 134 and 135 and then converge to generate a plume spray with a predetermined initial speed. The predetermined initial speed is less than 10 m/s, ranges from 1 m/s to 5 m/s or ranges from 0.5 m/s to 2 m/s, and at least 50% of droplets in the generated plume spray have an average particle size ranging from 1 um to 10 um, and preferably have an average particle size ranging from 2 um to 5 um. The plume spray of the liquid preparation containing nicotine substances with such an initial speed and particle size can be more conducive to absorption of the human and improve bioavailability.

In some embodiments, the pushing force in the step 501 is applied to the injector proximal end 127 by a thumb or other fingers. Since a human finger applying a pushing force is to gradually increase the force according to the force application habit, the bias spring 104 is configured to substantially offset the increasing part of the pushing force applied by the finger according to the human force application habit, so that in the process of step 501, the liquid conveying pipe 121 can maintain a substantially stable movement to the distal position, thereby maintaining a stable pressure in the liquid channel throughout the entire process or at least most of the duration, and maintaining the predetermined nebulizing pressure at the nebulizing spray nozzle. In some embodiments, the nebulizing device 100 is constructed as that, the plume spray formed in step 501 maintains the above-mentioned particle size or initial speed for at least 40% to 80% continuous time period of the duration, and preferably maintains the above-mentioned particle size or initial speed for 60% to 80% continuous time period of the duration, or maintains the above-mentioned particle size or initial speed for a longer time period, thereby effectively improving absorption efficiency and bioavailability.

In addition, due to a damping effect generated by the bias spring 104, in step 501, a time for the liquid conveying pipe 121 to move from the proximal position to the distal position is not less than a predetermined nebulizing time, for example, ranges from 1 second to 3 seconds, or is substantially equal to a single inhalation time of a normal breathing rhythm of a human. According to the construction of the nebulizing device 100, under common use, the duration of the plume spray is substantially equal to a single inhalation time, so as to avoid waste of liquid to be conveyed and improve the bioavailability. On the other hand, since the duration of the plume spray is substantially equal to the single inhalation time of a breathing rhythm, a problem of difficulty in breathing coordination and cooperation of a user caused by plume spray rapid generation devices such as an MDI is avoided.

In step 502, after the liquid conveying pipe 121 moves to the distal position shown in FIG. 2, the pushing force applied to the injector proximal end 127 is removed. Therefore, under an action of the bias spring 104, the liquid conveying pipe 121 moves from the distal position shown in FIG. 2 back to the proximal position shown in FIG. 1. In this process, with the movement of the liquid conveying pipe 121, a pressure in the liquid chamber 124 is gradually reduced, so that the liquid preparation containing nicotine substances in the reservoir 120 can be conveyed to the liquid chamber 124 through the liquid conveying pipe 121 based on a pressure difference between the liquid chamber 124 and the reservoir 120. After the liquid conveying pipe moves back to the proximal position, the user can choose to repeat step 501, to perform a next round of conveying the liquid preparation containing nicotine substances for pulmonary inhalation.

Compared with the conventional mechanical nebulizing device, the nebulizing device of this application overcomes prejudice of the existing technology, does not require an energy storage device and an instantaneous energy release switch, realizes real-time release of kinetic energy and real-time adaptive adjustment of nebulizing kinetic energy, avoids an impact of the instantaneous release of energy on the interior of the device and the irritable stimulation to senses of the user, achieves a good effect on an nebulizing effect of the liquid preparation and utilization of cooperation with the breathing of the user, and achieves an unexpected technical effect. In addition, the structure of the nebulizing device of this application is simplified, to reduce costs; no impact is generated from the instantaneous release of energy, and the use process is relatively mild; and the device is easy to operate and more acceptable to the user.

The nebulizing device according to the embodiments of this application is particularly suitable for delivering a nicotine preparation for pulmonary inhalation or other aqueous preparations. By means of delivery manners such as manual pushing, the plume spray of the nicotine preparation formed by the nebulizing device can achieve relatively high delivery efficiency and bioavailability with a lower nicotine concentration, and maintain the delivery efficiency substantially stable throughout the entire delivery process. In some embodiments, droplets of the preparation with an average particle size ranging from 1 um to 10 um can be delivered, which is sufficient to reach the alveolus region and can be retained in the alveolus region to avoid carrying excess unabsorbed nicotine in exhaled air.

In some embodiments, the nicotine may be nicotine free base or a nicotine derivative. The nicotine derivative may be any nicotine-like molecule capable of binding to a nicotinic acetylcholine receptor. Suitable nicotine-like molecules include acetylcholine, choline, epibatidine, lobeline, varenicline (varenicl ine), and cytisine. The liquid preparation may contain nicotine in an amount ranging from 0 mg/ml to 20 mg/ml, from 2 mg/ml to 20 mg/ml, from 2 mg/ml to 15 mg/ml, from 1 mg/ml to 8 mg/ml, or from 1.5 mg/ml to 6 mg/ml.

In some embodiments, a pH value of the nicotine liquid preparation delivered by the nebulizing device ranges from 7.0 to 12. Nicotine can be added to the preparation without pH adjustment, so that the nicotine exists primarily in an uncharged form equivalent to a pH value of about 10 (>99% uncharged). Since charged nicotine molecules (most nicotine at pH<8) are not volatile and diffuse slowly through active substances of a lung surface in the form of dissolved salt, uncharged nicotine can move into the gas phase and diffuse rapidly across membranes into the bloodstream. Therefore, nicotine at a pH value of 10 is preferentially delivered to the lung, which allows the delivered nicotine to participate in the substance exchange of the lung to the greatest extent.

The nicotine liquid preparation described herein may include at least one buffer system, and although the nicotine may be considered a buffer, the preparation also includes another buffer. The inventor of this application has found experimentally that when the liquid preparation is exposed to the air, the pH value of the liquid preparation gradually drops out of the optimal pH range of 10 due to the influence of CO₂ in the air. Therefore, for a preparation that is used for multiple times after opening and requires a guaranteed shelf life of three to nine months, addition of another buffer system is beneficial.

The nicotine liquid preparation described in this application includes at least one preservative or antioxidant, such as benzalkonium chloride or disodium EDTA, to ensure security and stability of the preparation during storage and use.

In some embodiments of this application, the liquid preparation does not include glycerol and propylene glycol.

The nicotine liquid preparation described in this application may include at least one inorganic or organic salt, such as sodium chloride, sodium citrate, or sodium sulfate. The inventor of this application has found experimentally that increasing the concentration of salt facilitates generation of droplets with a smaller particle size. The liquid preparation may contain 0.3% to 3% vol/vol of such salt.

The nicotine liquid preparation described in this application may be ethanol-free. Because ethanol reduces the viscosity and surface tension of the aqueous solution, which is not conducive to generation of small droplets, and ethanol volatilizes very quickly at a lower concentration, which is not conducive to the use and storage stability of the preparation.

The liquid preparation of this application may not contain propellant. In this application, the term "propellant" refers to a compound with a boiling point ranging from - 100°C to +30°C, a density ranging from 1.2 g/cm³ to 1.5 g/cm³, and a vapor pressure ranging from 40 psig to 80 psig that is non-flammable and non-toxic to human inhalation, such as hydrofluoridealkane (HFA) propellant. In this application, the propellant is a chemical substance used to generate a pressurized gas that is then used to cause fluid to move when the pressure is released.

The liquid preparation of this application may include: 2 mg/ml to 20 mg/ml of nicotine, 0.3% to 5% (v/v) of organic or inorganic salt such as NaCl; and at least one buffer system, for example, a Tris-HCl buffer system, and at least one preservative, for example, benzalkonium chloride. A pH value of the liquid preparation ranges from 7.0 to 12.

The liquid preparation of this application may further include one or more additives. For example, the liquid preparation may include flavoring components. Suitable flavoring components include those flavoring components typically added to tobacco products, for example, menthol, fruity, coffee, tobacco, or sweet components. Concentration of the flavoring component is selected to cause the component not to affect nicotine gas release or a droplet size. Alternatively or additionally, the liquid preparation may include substances that enhance throat impact, for example, citric acid. Alternatively or additionally, the liquid preparation may include a colouring agent, for example, caramel. Alternatively or additionally, the liquid preparation may include a sweetening agent, for example, glucose. Alternatively or additionally, the liquid preparation may include an antioxidant, for example, vitamin E. Alternatively or additionally, the liquid preparation may include a surfactant, for example, phospholipid (for example, oleic acid, lecithin, spans, and PVP). Alternatively or additionally, the liquid preparation may include a pH adjusting agent that will dissociate in solution, for example, HCl.

The above is a summary of this application, and there may be cases of simplification, generalization, and omission of details, so that a person skilled in the art should realize that this part is only illustrative, and is not intended to limit the scope of this application in any way. This summary is not intended to identify key features or essential features of the claimed subject matter, nor is it intended to be used as an aid in determining the scope of the claimed subject matter.

Although several components or subcomponents of the nebulizing device are mentioned in the foregoing detailed description, such division is merely an example but not mandatory. Actually, according to the embodiments of this application, the features and functions of two or more components described above may be embodied in one component. On the contrary, the features or functions of one component described above may further be divided and specified by a plurality of components.

A person skilled in the art may understand and implement other changes to the disclosed embodiments by studying the specification, the disclosed contents, the accompanying drawings, and the appended claims. In the claims, the word "comprising" does not exclude other elements and steps, and the words "a" and "an" do not exclude a plural form. In a practical application of this application, one component may perform the functions of several technical features recited in the claims. Any reference numeral in the claims shall not be construed as limiting the scope.

## Claims

1. A nebulizing device (100), comprising:
a hollow housing (101), wherein the hollow housing (101) comprises a cavity (110), and the cavity (110) comprises a liquid channel (111) located inside the cavity (110) and a nebulizing spray nozzle (103) located at a distal end of the liquid channel (111), wherein the nebulizing spray nozzle (103) comprises a plurality of fluid outlets (134, 135); and
an injector (102), wherein the injector (102) is at least partially accommodated in the cavity (110), and coupled to the hollow housing (101) through a bias spring (104) and the injector (102) comprises:
a reservoir (120), configured to accommodate liquid;
a liquid conveying pipe (121), comprising a liquid inlet and a liquid outlet (123) arranged opposite to each other, wherein the liquid inlet is located in the reservoir (120), the liquid outlet (123) is located in the liquid channel (111), and the liquid outlet is provided with a one-way valve member (125), the one-way valve member (125) and the nebulizing spray nozzle (103) jointly define a liquid chamber (124), the liquid can flow into the liquid chamber (124) through the one-way valve member (125) but cannot flow out of the liquid chamber (124) in an opposite direction, and the liquid conveying pipe (121) is configured to operatively convey the liquid accommodated in the reservoir (120) into the liquid chamber (124); and
an injector proximal end (127), coupled to the liquid conveying pipe (121) and configured to operatively receive a manually applied pushing force, wherein under an action of the pushing force and the bias spring (104), the liquid conveying pipe (121) is movable between a proximal position in the cavity and a distal position relatively closer to the distal end; and when the liquid conveying pipe (121) is at the distal position, the bias spring (104) is in a compressed state, wherein
when the pushing force is removed from the injector proximal end (127), the bias spring (104) is capable to drive the liquid conveying pipe (121) to move from the distal position to the proximal position, so as to convey the liquid from the reservoir into the liquid chamber (124);
when the injector proximal end (127) receives the pushing force, the pushing force is capable to compress the bias spring (104) to drive the liquid conveying pipe (121) to move from the proximal position to the distal position, so as to form a predetermined nebulizing pressure at the nebulizing spray nozzle (103), so that the liquid in the liquid chamber (124) can flow out of the liquid channel (111) through the nebulizing spray nozzle (103).

2. The nebulizing device (100) according to claim 1, wherein the predetermined initial speed is less than 10 m/s, preferably wherein the predetermined initial speed ranges from 1 m/s to 5 m/s.

3. The nebulizing device (100) according to claim 1, wherein the bias spring (104) is configured to, when the pushing force pushes the liquid conveying pipe (121) to move from the proximal position to the distal position, generate a resistance in an opposite direction to the pushing force, and the resistance is used for substantially offsetting an increased pushing force, so that the predetermined nebulizing pressure is maintained at the nebulizing spray nozzle (103).

4. The nebulizing device (100) according to claim 1 to 3, wherein the bias spring (104) is configured to generate a resistance when the pushing force pushes the liquid conveying pipe (121) to move from the proximal position to the distal position, and the resistance and a liquid resistance in the liquid channel (111) cause a time for the liquid conveying pipe (121) to move from the proximal position to the distal position to be not less than a predetermined nebulizing time, preferably wherein the predetermined nebulizing time ranges from 0.5 seconds to 5 seconds.

5. The nebulizing device (100) according to claim 4, wherein the resistance causes the time for the liquid conveying pipe (121) to move from the proximal position to the distal position to be substantially equal to a single inhalation time of a normal breathing rhythm of a human.

6. The nebulizing device (100) according to any one of claims 1 to 5, wherein the pushing force is manually applied and wherein a magnitude of the pushing force ranges from 10 N to 70 N.

7. The nebulizing device (100) according to any one of claims 1 to 6, wherein the nebulizing device further comprises a distal stopper, and the distal stopper is arranged in the hollow housing (101) and is configured to, when the liquid conveying pipe (121) is at the distal position, prevent the liquid conveying pipe (121) from moving distally.

8. The nebulizing device (100) according to any one of claims 1 to 7, wherein the nebulizing device further comprises a proximal stopper (105), and the proximal stopper (105) is arranged in the hollow housing (101) and is configured to, when the liquid conveying pipe (121) is at the proximal position, prevent the liquid conveying pipe (121) from moving proximally.

9. The nebulizing device (100) according to any one of claims 1 to 8, wherein a cross-sectional area of the plurality of fluid outlets (134, 135) of the nebulizing spray nozzle (103) ranges from 20 um² to 1000 um².

## Patentansprüche

1. Eine Vernebelungsvorrichtung (100), umfassend:
ein hohles Gehäuse (101), wobei das hohle Gehäuse (101) einen Hohlraum (110) umfasst, und der Hohlraum (110) einen innerhalb des Hohlraums (110) angeordneten Flüssigkeitskanal (111) sowie eine an einem distalen Ende des Flüssigkeitskanals (111) angeordnete Vernebelungsdüse (103) umfasst, wobei die Vernebelungsdüse (103) mehrere Fluidauslässe (134, 135) umfasst; und
einen Injektor (102), wobei der Injektor (102) zumindest teilweise im Hohlraum (110) untergebracht und über eine Vorspannfeder (104) mit dem hohlen Gehäuse (101) verbunden ist und der Injektor (102) Folgendes umfasst:
einen Vorratsbehälter (120), der zur Aufnahme von Flüssigkeit ausgelegt ist;
eine Flüssigkeitsförderleitung (121), die einen Flüssigkeitseinlass und einen Flüssigkeitsauslass (123) umfasst, welche einander gegenüberliegend angeordnet sind, wobei sich der Flüssigkeitseinlass im Vorratsbehälter (120) befindet, der Flüssigkeitsauslass (123) sich im Flüssigkeitskanal (111) befindet, und der Flüssigkeitsauslass mit einem Rückschlagventilelement (125) versehen ist, wobei das Rückschlagventilelement (125) und die Vernebelungsdüse (103) gemeinsam eine Flüssigkeitskammer (124) ausbilden, wobei die Flüssigkeit durch das Rückschlagventilelement (125) in die Flüssigkeitskammer (124) fließen kann, jedoch nicht in entgegengesetzter Richtung aus der Flüssigkeitskammer (124) herausfließen kann, und die Flüssigkeitsförderleitung (121) dazu ausgebildet ist, die im Vorratsbehälter (120) aufgenommene Flüssigkeit im Betrieb in die Flüssigkeitskammer (124) zu fördern; und
ein proximales Injektorende (127), das mit der Flüssigkeitsförderleitung (121) verbunden und dazu ausgebildet ist, eine manuell ausgeübte Druckkraft im Betrieb aufzunehmen, wobei unter der Wirkung der Druckkraft und der Vorspannfeder (104) die Flüssigkeitsförderleitung (121) zwischen einer proximalen Position im Hohlraum und einer distalen Position, die relativ näher am distalen Ende liegt, bewegbar ist; und wobei dann, wenn sich die Flüssigkeitsförderleitung (121) in der distalen Position befindet, die Vorspannfeder (104) sich in einem zusammengedrückten Zustand befindet, wobei
dann, wenn die Druckkraft vom proximalen Injektorende (127) weggenommen wird, die Vorspannfeder (104) in der Lage ist, die Flüssigkeitsförderleitung (121) anzutreiben, sich von der distalen Position in die proximale Position zu bewegen, um die Flüssigkeit aus dem Vorratsbehälter in die Flüssigkeitskammer (124) zu fördern;
dann, wenn das proximale Injektorende (127) die Druckkraft aufnimmt, diese Druckkraft in der Lage ist, die Vorspannfeder (104) zusammenzudrücken, um die Flüssigkeitsförderleitung (121) von der proximalen Position in die distale Position zu bewegen, um an der Vernebelungsdüse (103) einen vorbestimmten Vernebelungsdruck zu erzeugen, sodass die Flüssigkeit in der Flüssigkeitskammer (124) durch den Flüssigkeitskanal (111) und aus der Vernebelungsdüse (103) ausströmen kann.

2. Vernebelungsvorrichtung (100) nach Anspruch 1, wobei die vorbestimmte Anfangsgeschwindigkeit weniger als 10 m/s beträgt, vorzugsweise wobei die vorbestimmte Anfangsgeschwindigkeit im Bereich von 1 m/s bis 5 m/s liegt.

3. Vernebelungsvorrichtung (100) nach Anspruch 1, wobei die Vorspannfeder (104) dazu ausgebildet ist, dann, wenn die Druckkraft die Flüssigkeitsförderleitung (121) von der proximalen Position in die distale Position bewegt, einen Widerstand in einer der Druckkraft entgegengesetzten Richtung zu erzeugen, wobei der Widerstand dazu dient, eine erhöhte Druckkraft im Wesentlichen auszugleichen, sodass der vorbestimmte Vernebelungsdruck an der Vernebelungsdüse (103) aufrechterhalten wird.

4. Vernebelungsvorrichtung (100) nach einem der Ansprüche 1 bis 3, wobei die Vorspannfeder (104) dazu ausgebildet ist, dann einen Widerstand zu erzeugen, wenn die Druckkraft die Flüssigkeitsförderleitung (121) dazu bewegt, sich von der proximalen Position in die distale Position zu bewegen, und wobei der Widerstand sowie ein Flüssigkeitswiderstand im Flüssigkeitskanal (111) bewirken, dass die Zeit, die die Flüssigkeitsförderleitung (121) benötigt, um sich von der proximalen Position in die distale Position zu bewegen, nicht kürzer als eine vorbestimmte Vernebelungszeit ist, wobei die vorbestimmte Vernebelungszeit vorzugsweise im Bereich von 0,5 Sekunden bis 5 Sekunden liegt.

5. Vernebelungsvorrichtung (100) nach Anspruch 4, wobei der Widerstand bewirkt, dass die Zeit, die die Flüssigkeitsförderleitung (121) benötigt, um sich von der proximalen Position in die distale Position zu bewegen, im Wesentlichen einer einzelnen Einatmungszeit eines normalen Atemrhythmus eines Menschen entspricht.

6. Vernebelungsvorrichtung (100) gemäß einem der Ansprüche 1 bis 5, wobei die Druckkraft manuell ausgeübt wird und wobei die Größe der Druckkraft im Bereich von 10 N bis 70 N liegt.

7. Vernebelungsvorrichtung (100) nach einem der Ansprüche 1 bis 6, wobei die Vernebelungsvorrichtung ferner einen distalen Anschlag umfasst, und der distale Anschlag im hohlen Gehäuse (101) angeordnet und dazu ausgebildet ist, dann, wenn sich die Flüssigkeitsförderleitung (121) in der distalen Position befindet, eine Bewegung der Flüssigkeitsförderleitung (121) in distaler Richtung zu verhindern.

8. Vernebelungsvorrichtung (100) nach einem der Ansprüche 1 bis 7, wobei die Vernebelungsvorrichtung ferner einen proximalen Anschlag (105) umfasst, und der proximale Anschlag (105) im hohlen Gehäuse (101) angeordnet und dazu ausgebildet ist, dann, wenn sich die Flüssigkeitsförderleitung (121) in der proximalen Position befindet, eine Bewegung der Flüssigkeitsförderleitung (121) in proximaler Richtung zu verhindern.

9. Vernebelungsvorrichtung (100) nach einem der Ansprüche 1 bis 8, wobei eine Querschnittsfläche der mehreren Fluidauslässe (134, 135) der Vernebelungsdüse (103) im Bereich von 20 µm² bis 1000 µm² liegt.

## Revendications

1. Dispositif de nébulisation (100), comprenant :
un boîtier creux (101), dans lequel le boîtier creux (101) comprend une cavité (110), et ladite cavité (110) comportant un canal de liquide (111) situé à l'intérieur de la cavité (110) et une buse de nébulisation (103) située à une extrémité distale du canal de liquide (111), ladite buse de nébulisation (103) comportant une pluralité de sorties de fluide (134, 135) ; et
un injecteur (102), dans lequel l'injecteur (102) est au moins partiellement logé dans la cavité (110), et est couplé au boîtier creux (101) par l'intermédiaire d'un ressort de rappel (104), et l'injecteur (102) comprend :
un réservoir (120), configuré pour contenir du liquide ;
un tuyau d'acheminement de liquide (121), comprenant une entrée de liquide et une sortie de liquide (123) disposées en vis-à-vis l'une de l'autre, l'entrée de liquide étant située dans le réservoir (120), la sortie de liquide (123) étant située dans le canal de liquide (111), et la sortie de liquide étant munie d'un élément de clapet anti-retour (125), l'élément de clapet anti-retour (125) et la buse de nébulisation (103) définissent conjointement une chambre à liquide (124), le liquide peut s'écouler dans la chambre à liquide (124) à travers l'élément de clapet anti-retour (125) mais ne peut pas s'écouler hors de la chambre à liquide (124) dans le sens inverse, et le tuyau d'acheminement de liquide (121) est configuré pour acheminer de manière fonctionnelle le liquide contenu dans le réservoir (120) vers la chambre à liquide (124) ; et
une extrémité proximale d'injecteur (127), couplée au tuyau d'acheminement de liquide (121) et configurée pour recevoir de manière fonctionnelle une force de poussée appliquée manuellement, dans laquelle, sous l'action de la force de poussée et du ressort de rappel (104), le tube d'acheminement de liquide (121) est mobile entre une position proximale dans la cavité et une position distale relativement plus proche de l'extrémité distale ; et lorsque le tube d'acheminement de liquide (121) se trouve dans la position distale, le ressort de rappel (104) est dans un état comprimé, dans lequel
lorsque la force de poussée est retirée de l'extrémité proximale (127) de l'injecteur, le ressort de rappel (104) est capable d'entraîner le tube d'acheminement de liquide (121) à se déplacer de la position distale vers la position proximale, de manière à acheminer le liquide du réservoir vers la chambre à liquide (124) ;
lorsque l'extrémité proximale (127) de l'injecteur reçoit la force de poussée, celle-ci est capable de comprimer le ressort de rappel (104) pour entraîner le tube d'acheminement de liquide (121) à se déplacer de la position proximale vers la position distale, de manière à former une pression de nébulisation prédéterminée au niveau de la buse de nébulisation (103), de sorte que le liquide contenu dans la chambre à liquide (124) puisse s'écouler hors du canal de liquide (111) à travers la buse de nébulisation (103).

2. Dispositif de nébulisation (100) selon la revendication 1, dans lequel la vitesse initiale prédéterminée est inférieure à 10 m/s, de préférence dans lequel la vitesse initiale prédéterminée est comprise entre 1 m/s et 5 m/s.

3. Dispositif de nébulisation (100) selon la revendication 1, dans lequel le ressort de rappel (104) est configuré pour, lorsque la force de poussée pousse le tuyau d'acheminement de liquide (121) à se déplacer de la position proximale vers la position distale, générer une résistance dans une direction opposée à la force de poussée, et la résistance est utilisée pour compenser sensiblement une force de poussée accrue, de sorte que la soit maintenue au niveau de la buse de nébulisation (103).

4. Dispositif de nébulisation (100) selon l'une des revendications 1 à 3, dans lequel le ressort de rappel (104) est configuré pour générer une résistance lorsque la force de poussée pousse le tube d'acheminement de liquide (121) à se déplacer de la position proximale vers la position distale, et la résistance ainsi qu'une résistance au liquide dans le canal de liquide (111) font en sorte que le temps nécessaire au tube d'acheminement de liquide (121) passe de la position proximale à la position distale soit au moins égale à un temps de nébulisation prédéterminé, de préférence le temps de nébulisation prédéterminé étant compris entre 0,5 seconde et 5 secondes.

5. Dispositif de nébulisation (100) selon la revendication 4, dans lequel la résistance fait en sorte que le temps nécessaire au tube d'acheminement de liquide (121) pour se déplacer de la position proximale à la position distale soit sensiblement égal à la durée d'une seule inspiration dans le cadre d'un rythme respiratoire normal chez l'être humain.

6. Dispositif de nébulisation (100) selon l'une quelconque des revendications 1 à 5, dans lequel la force de poussée est appliquée manuellement et dans lequel l'amplitude de la force de poussée est comprise entre 10 N et 70 N.

7. Dispositif de nébulisation (100) selon l'une quelconque des revendications 1 à 6, dans lequel le dispositif de nébulisation comprend en outre une butée distale, et la butée distale est disposée dans le boîtier creux (101) et est configurée pour, lorsque le tube d'acheminement de liquide (121) se trouve en position distale, empêcher le tube d'acheminement de liquide (121) de se déplacer vers l'extrémité distale.

8. Dispositif de nébulisation (100) selon l'une quelconque des revendications 1 à 7, dans lequel le dispositif de nébulisation comprend en outre une butée proximale (105), et dans lequel la butée proximale (105) est disposée dans le boîtier creux (101) et est configurée pour, lorsque le tube d'acheminement de liquide (121) se trouve en position proximale, empêcher le tube d'acheminement de liquide (121) de se déplacer en direction proximale.

9. Dispositif de nébulisation (100) selon l'une quelconque des revendications 1 à 8, dans lequel l'aire de section transversale de la pluralité de sorties de fluide (134, 135) de la buse de nébulisation (103) est comprise entre 20 µm² et 1 000 µm².
